# FASCICULE DE BREVET EUROPEEN

(11) **EP 1 791 486 B1**
(45) Date de publication et mention de la délivrance du brevet: **18.11.2015**
(21) Numéro de dépôt: 05779716.9
(22) Date de dépôt: 01.07.2005
(51) Int. Cl.: A61B 19/02, B65D 65/42, A61L 2/26

(54) **MATERIAU D'EMBALLAGE DE STERILISATION RENFORCE ET EMBALLAGE LE COMPRENANT**
VERSTÄRKTES STERILISATIONSVERPACKUNGSMATERIAL UND VERPACKUNG AUS DIESEM MATERIAL
REINFORCED STERILISING PACKAGING MATERIAL AND PACKAGING CONTAINING SAID MATERIAL

(30) Priorité: 02.07.2004 FR 0407343
(43) Date de publication de la demande: 06.06.2007
(73) Titulaire: Arjowiggins, 92100 Boulogne Bilancort (FR)
(72) Inventeur: PARIS-JOLLY, Agnès, 38140 La Murette (FR); SIMON, Christophe, 66480 Maureillas Las Illas (FR)
(74) Mandataire: Tanty, François
(86) Numéro de dépôt international: PCT/FR2005/001678
(87) Numéro de publication internationale: WO 2006/013255

(56) Documents cités:
- WO-A-00/21745
- WO-A-96/16562
- US-A- 5 730 530

## Description

La présente invention concerne un matériau d'emballage de stérilisation pour les dispositifs médicaux devant être stérilisés, ainsi que l'emballage de stérilisation lui-même.

On connaît déjà des emballages de stérilisation pour des dispositifs médicaux devant être stérilisés, notamment d'instruments ou de matériels réutilisables, comme par exemple les sondes, scalpels, pinces, ciseaux, aiguilles.

Ces emballages se présentent alors sous la forme d'un sachet, d'une pochette, d'une gaine ou d'un blister ou encore sous forme d'emballage rigide. Les matériaux pour ces emballages sont des feuilles à base de fibres uniquement de cellulose (sans fibres synthétiques) qui sont fabriquées et commercialisées en Europe par la société ARJOWIGGINS sous les marques ETHYPEL ® et PROPYPEL ® ou ARJOPEEL ™. Ces feuilles ont une barrière bactérienne élevée mais ont une résistance mécanique qui peut s'avérer insuffisante même si elles ont été traitées en surface par un agent renforçant comme l'amidon, le polyalcool de vinyle ou un latex acrylique, lorsque l'on souhaite emballer des objets lourds ou contondants.

Ces emballages peuvent également se présenter sous forme de feuille pliable autour des objets ou dispositifs à stériliser, tels que du matériel chirurgical réutilisable comme par exemple un kit orthopédique. Ce sont des feuilles de papier souples ou de non-tissés qui permettent la stérilisation dudit matériel et assurent leur maintien à l'état stérile, jusqu'au moment de l'opération chirurgicale, pour laquelle on déplie la feuille, le dépliage pouvant se faire soit dans un sas avant d'entrer dans la salle d'opération, soit uniquement en salle d'opération. Ces feuilles peuvent être des feuilles papetières renforcées par des fibres synthétiques en mélange avec les fibres de cellulose. Par exemple de telles feuilles comportant des fibres synthétiques de polyester sont vendues par la société ARJOWIGGINS sous la marque STERISHEET ®. A grammage équivalent, ces feuilles dites renforcées ont une résistance mécanique supérieure aux feuilles purement cellulosiques mais en revanche leur barrière bactérienne est un peu moins élevée. L'emballage rigide est constitué d'un récipient en général en plastique et thermoformé qui contiendra les dispositifs médicaux à stériliser puis qui sera fermé par un opercule qui peut être une feuille de papier barrière aux micro-organismes et scellable.

L'emballage souple ou semi-rigide sous forme d'un sachet, d'une pochette, d'une gaine ou d'un blister est un emballage constitué d'une partie qui peut être en matière synthétique et d'une feuille de papier ou de non-tissé ayant une perméabilité spécifique, scellées entre elles sur un certain périmètre selon la forme voulue pour l'emballage, une ouverture plus ou moins grande étant laissée afin de pouvoir introduire les objets.

Les objets à stériliser sont placés à l'intérieur de l'emballage puis on scelle complètement ledit emballage. La partie en matière synthétique peut être un film thermoplastique comme le polyéthylène ou le polypropylène. Ce film est en général imperméable aux gaz et à la vapeur d'eau et de plus transparent afin de voir le contenu de l'emballage. A la place du film plastique, on peut utiliser aussi une feuille semblable à la feuille de papier qui a une perméabilité spécifique ou une feuille de papier enduite d'un produit scellant comme une couche de polyéthylène extrudé ou du poly(acétate de vinyle).

Dans le cas d'un blister, on utilise un film plastique souple qui est thermoformé selon la forme du dispositif à emballer.

La feuille, utilisée dans ces emballages rigides ou souples, doit avoir une perméabilité spécifique qui la rend barrière aux micro-organismes mais qui permet de laisser passer les agents stérilisants afin de réaliser la stérilisation de l'emballage fermé et de son contenu par des méthodes de stérilisation utilisant comme agents stérilisants la vapeur d'eau ou des gaz stérilisants comme l'oxyde d'éthylène ou le formaldéhyde. L'emballage peut aussi être stérilisé par des rayonnements ionisants comme des rayonnements gamma ou bêta.

Dans la demande de brevet WO00/21745, qui est considérée comme l'état de la technique le plus proche à la présente invention, on a proposé un matériau ayant des caractéristiques mécaniques et des propriétés de barrières aux micro-organismes relativement élevées. Ce matériau consiste en au moins deux feuilles d'emballage de stérilisation liées entre elles par l'une de leurs faces de façon non réversible, notamment par contrecollage. Toutefois la conception de ce matériau d'emballage de stérilisation présente encore quelques inconvénients.

D'une part, la réalisation du matériau lui-même nécessite un processus de fabrication relativement lourd et complexe, ainsi qu'une consommation additionnelle de produits de scellage.

Ceci génère de ce fait un coût de revient encore trop élevé et conduit à un matériau de grammage relativement élevé, n'allant pas dans le sens des recommandations selon la Directive 2004/CE/12 de l'Union Européenne sur les emballages et déchets d'emballage préconisant une diminution du poids des emballages.

D'autre part, certaines des propriétés de l'emballage de stérilisation obtenu selon le brevet WO00/21745 peuvent être jugés insuffisantes au regard d'applications médicales bien spécifiques.

En particulier, l'une des propriétés recherchées est que la feuille soit résistante à la déchirure. En effet, comme elle est destinée à faire des emballages qui vont contenir des objets qui peuvent être lourds ou contondants, elle risque d'être déchirée ou transpercée par ces objets lors de la manipulation desdits emballages. Dans le cas d'un emballage de stérilisation scellable, on recherche une résistance moyenne à la déchirure amorcée supérieure à 300 mN (mesurée selon la norme européenne EN 21974). Toutefois, dans certaines applications, il est préférable que cette résistance à la déchirure amorcée soit bien supérieure, d'au moins 1900 mN (mesurée selon la norme européenne EN 21974).

Une autre des propriétés recherchées est que la feuille constitue une barrière efficace aux bactéries ou autres micro-organismes pour maintenir la stérilité de l'emballage c'est-à-dire qu'il ne faut pas que les micro-organismes puissent pénétrer à l'intérieur de l'emballage après la stérilisation. Il faut donc que la moyenne des diamètres les plus élevés des pores ne soit pas trop importante et qu'il n'y ait pas de pores avec un diamètre trop élevé. Cette propriété barrière peut être caractérisée par l'efficacité en filtration bactérienne, nommée couramment sous les initiales de sa terminologie anglaise BFE (Bacterial Filtration Efficiency) ; elle est exprimée en pourcentage qui représente le pourcentage de bactéries arrêtées par la feuille. Dans le cas d'un emballage de stérilisation scellable, on recherche une BFE d'au moins 85 %.

Toutefois, il n'est pas rare d'avoir besoin, dans certains types d'emballage, de niveau de protection bactérienne bien supérieur, notamment possédant un BFE d'au moins 95 %.

Jusqu'à maintenant, la coexistence de ces deux propriétés à des niveaux élevés au sein du même emballage s'avère difficile.

En effet, dans l'art antérieur, les solutions adoptées correspondent toujours à un compromis entre la recherche d'une bonne résistance mécanique, notamment à la déchirure, et un bon effet barrière aux bactéries ou autres micro-organismes. L'obtention d'une valeur élevée pour l'une de ces propriétés entraînant alors une valeur relativement faible pour l'autre de ces propriétés.

Par conséquent il s'avère que les emballages de stérilisation disponibles actuellement sur le marché ne répondent pas toujours aux besoins des utilisateurs dans ce domaine ni aux Directives de l'Union Européenne préconisant une diminution du poids des emballages.

Le but de la présente invention est donc de fournir un matériau d'emballage de stérilisation qui présente des niveaux élevés à la fois en ce qui concerne sa résistance à la déchirure amorcée et son effet barrière aux micro-organismes tout en ayant un grammage le plus bas possible.

La Demanderesse a trouvé qu'avec des fibres synthétiques de type polyamide incorporées au sein de structures fibreuses on obtient un matériau qui répond au problème posé.

L'invention fournit ainsi un matériau d'emballage de stérilisation selon la revendication 1.

La feuille est formée d'une seule couche de fibres (monocouche ou monojet).

On entend par " résistance moyenne ", la moyenne arithmétique de la mesure de la résistance faite selon une première direction de la feuille et de la mesure de la résistance faite selon la direction perpendiculaire à cette première direction. Cette première direction correspond à une orientation privilégiée des fibres lorsqu'il y en a une, généralement il s'agit de la direction d'orientation des fibres selon leur sens long ; ce sens correspond au sens marche de la machine qui a servi à fabriquer la feuille, la direction perpendiculaire correspond donc au sens travers du sens marche de la machine. Dans le cas où les fibres n'ont pas d'orientation privilégiée, on réalise des mesures selon deux directions perpendiculaires.

De préférence, ladite feuille a un grammage inférieur ou égal à 90 g/m², mesuré selon la norme internationale ISO 536.

Selon un cas particulier de l'invention, les fibres de polyamide sont choisies parmi les fibres de polyamide 6, de polyamide 6-6, de polyamide 6-9 et les fibres de polyamide 6-10 et leurs mélanges. De préférence, les fibres de polyamide sont des fibres de polyamide 6-6. Ces fibres peuvent être de type bi-composants.

De préférence encore lesdites fibres de polyamide ont une longueur supérieure ou égale à 5mm. Leur longueur peut être par exemple comprise entre 5 et 25mm.

De préférence encore lesdites fibres de polyamide ont un titre moyen supérieur à 1,5 dtex.

Selon un cas particulier de l'invention, lesdites fibres de polyamide sont des fibres coupées.

Selon un autre cas particulier de l'invention, lesdites fibres de polyamide sont des fibres continues (filaments).

De préférence lesdites fibres de polyamide comportent des anti-oxydants leur conférant une résistance à l'irradiation.

Ladite composition fibreuse comporte en masse des fibres de cellulose raffinées, de préférence entre 25 et 70 degrés

Schoepper-Riegler, plus préférentiellement ayant un degré Schoepper-Riegler supérieur ou égal à 30 et inférieur à 60, et un liant papetier, ou ladite composition fibreuse comporte comme fibres cellulosiques une pâte fluff.

De préférence, ladite feuille fibreuse comporte en surface un liant de renforcement, en particulier un liant polymère de base acrylique ou encore un copolymère styrène-butadiène. De préférence la quantité dudit liant est comprise entre 10 et 35 g/m² en poids sec.

Selon un cas particulier de l'invention, ledit matériau a été assoupli, notamment (micro)crêpé ou flexé.

Par ailleurs, ledit matériau peut comporter un produit de scellage sur au moins l'une de ses faces. Il peut comporter sur au moins l'une de ses faces soit un produit thermoscellable, comme par exemple une cire microcristalline, en particulier de polyoléfine, un adhésif de type EVA (polymère EthylVinylAcétate), un adhésif de type EAA (Ethylène Acide Acétique), soit un produit scellable à froid comme par exemple le caoutchouc naturel.

Selon un cas particulier de l'invention, la feuille fibreuse dudit matériau comprend une quantité de fibres de polyamide supérieure à 10 % en poids par rapport au poids total en fibres. Selon un cas particulier de l'invention, les fibres de polyamide représente au moins 15 % en poids du poids total en fibres de ladite composition fibreuse.

Selon un autre cas particulier de l'invention, la composition fibreuse dudit matériau peut comporter comme fibres essentiellement des fibres synthétiques dont au moins 50%, de préférence au moins 60%, de fibres de polyamide, ce matériau peut être alors apte à la fois aux procédés de stérilisation par gaz, par irradiation, par vapeur d'eau, par plasma et par ozone. Ledit matériau peut être obtenu selon divers procédés connus.

Selon un cas particulier, ledit matériau peut être obtenu par un procédé voie humide, de type papetier. On peut utiliser une machine à papier à table plate, à table inclinée ou encore à forme ronde.

On peut par exemple réaliser une feuille selon l'invention sur une machine à papier avec des fibres de cellulose, éventuellement modifiée comme les fibres de rayonne issues du procédé sodique de la viscose ou des fibres de cellulose régénérées en milieu solvant comme celles commercialisées sous les marques Lyocell ® ou Tencel ®, en mélange avec les fibres PA et éventuellement d'autres fibres synthétiques telle que la pâte de fibres de polyéthylène (pâte PE), toutes ces fibres étant liées soit par thermoliage, soit par hydroliage ou soit par voie chimique grâce à l'ajout en masse d'un agent liant utilisé habituellement en papeterie. De plus, on peut appliquer un liant synthétique par un traitement de surface à l'aide d'une presse encolleuse ou par pulvérisation.

Selon un autre cas particulier de fabrication, ledit matériau peut être obtenu par un procédé voie sèche qui permet de former un non-tissé.

On peut en particulier fabriquer un non-tissé de type SMS (correspondant aux initiales de sa terminologie anglaise Spunbonded/MeltBlown/Spunbonded) qui est une feuille multicouche de fibres et filaments synthétiques usuellement employée dans le domaine des matériaux synthétiques pour emballages médicaux et hospitaliers. Ce non-tissé en particulier comporte des fibres polyamide continues. Dans le cas où on utilise de la pâte fluff, le matériau est formé par voie sèche, la pâte fluff et les fibres PA et éventuellement d'autres fibres synthétiques pouvant être liées par jet d'eau.

De façon générale, les fibres sont liées par des moyens connus tels que, par exemple, le thermoliage, l'hydroliage, les ultrasons.

Lors de sa fabrication, ledit matériau peut être assoupli notamment par un traitement à sec comme le (micro)crêpage ou le flexage, avant d'être revêtu du produit de scellage le cas échéant.

L'invention concerne aussi un emballage de stérilisation comprenant un matériau tel que décrit précédemment. Il peut s'agir de tout type d'emballage : sachet, pochette, container rigide, gaine, feuille d'emballage pour kits hospitaliers, blister ou encore de feuilles de protection de dispositifs médicaux.

L'invention sera mieux comprise à l'aide des exemples non limitatifs suivants.

### Exemple 1 comparatif :

On fabrique une feuille sur une machine à papier à table plate de la manière suivante :
On met en suspension en milieu aqueux des fibres de cellulose et des fibres synthétiques de PET (polyéthylènethéréphtalate). Les fibres de cellulose sont un mélange de fibres longues (résineux) et de fibres courtes, dans un ratio 3/2 en poids ; les fibres étant raffinées à 58 °SR. Les fibres PET ont une longueur de 6 mm et un titre de 1,7 dtex. A cette suspension, on ajoute 0,26 %, en poids sec de la composition totale de la feuille, d'un agent de résistance humide de type PAE (polyamine épichlorhydrine) et 1 %, en poids sec de la composition totale de la feuille, d'un amidon cationique comme agent de cohésion interne. On égoutte cette suspension sur la toile de la machine à papier pour former la feuille.

On imprègne la feuille en presse encolleuse d'un liant (agent de cohésion) synthétique, un polymère acrylique utilisé sous forme d'une dispersion aqueuse stabilisée (appelée couramment latex). Ce liant acrylique est présent à raison de 18 g/m² en poids sec. On sèche la feuille vers 120°C. La feuille a alors un grammage de 80 g/m².

### Exemples 2 à 6 selon l'invention.

Les feuilles sont réalisées selon le même mode de fabrication que celui à l'exemple 1, les fibres PET étant remplacées par des fibres de polyamide.

### Exemple 7 :

On réalise une feuille ne comprenant que des fibres synthétiques. On met en suspension, en milieu aqueux, une pâte de fibres synthétiques de polyéthylène (pâte PE ayant un rôle de liant) auxquelles on ajoute des fibres de polyamide ayant une longueur de 6 mm et un titre de 1,7 dtex. A cette suspension, on ajoute en masse un liant synthétique, un latex de copolymère(styrène-butadiène), à raison de 30 g/m² en poids sec.

On égoutte cette suspension sur la toile de la machine pour former la feuille.

On sèche la feuille vers 120 °C.

La feuille a alors un grammage de 80 g/m².

### Données et résultats des exemples :

Les données sur les composants des feuilles et les caractéristiques sont exprimées dans le tableau 1.

L'exemple 1 ne comprenant pas de fibres de polyamide a une résistance à la déchirure amorcée inférieure à 1900 mN alors que les exemples 2 à 6 répondent aux critères de l'invention.

### METHODES DE CARACTERISATION :

Les feuilles obtenues selon les exemples ont été caractérisées par les méthodes référencées ci-dessous.

Les mesures, sauf la BFE, ont été faites sur des échantillons conditionnés selon la norme européenne EN 20187 (équivalente à la norme ISO 187 : 1995) selon laquelle la température doit être maintenue à 23 °C et l'humidité relative à 50 %. Le grammage est déterminé selon la nonne internationale ISO 536.

La résistance moyenne (moyenne des mesures sens marche et sens travers de la machine) à la déchirure amorcée est mesurée selon la norme européenne EN 21974 et qui correspond à la norme internationale ISO 1974:1990 (méthode Elmendorf).

La perméabilité moyenne à l'air est mesurée selon la norme ISO 5636/3 (méthode Bendtsen).

La moyenne du diamètre des pores équivalent est mesurée selon la norme européenne EN 868-3- annexe B.

L'efficacité en filtration bactérienne BFE est déterminée selon la méthode publiée par l'association européenne EDANA sous la référence 180.0-89 de février 1996.

**TABLEAU 1**

| EXEMPLES | Exemple 1 comparatif | Exemple 2 | Exemple 3 | Exemple 4 | Exemple 5 | Exemple 6 | Exemple 7 |
|---|---|---|---|---|---|---|---|
| Quantité de fibres cellulosiques | 41 g/m² | 35 g/m² | 35 g/m² | 41 g/m² | 41 g/m² | 56 g/m² | 0 |
| Raffinage des fibres cellulosiques (°SR) | 58 | 62 | 59 | 58 | 60 | 30 | / |
| Quantité, nature - titre, longueur des fibres synthétiques | 21 g/m²,PET-l,7dtex, : 6mm | 30 g/m², PA 6,6 -1,7 dtex, 6mm | 30 g/m², PA 6,6 - 1,7 dtex, 6mm | 21 g/m², PA 6,6 - 1,7 dtex, 6mm | 21 g/m², PA 6,6 - 1,7 dtex, 6mm | 12g/m², PA 6,6-1,7 dtex, 6mm | 30 g/m² ,PA 6,6 - 1,7 dtex, 6mm 20 g/m² pâte PE |
| Quantité en sec et nature du liant synthétique de surface | 18 g/m², polymère acrylique Primal E1845 de Rhom&Haas | 15 g/m², polymère acrylique Acronal LA471S de BASF | 15 g/m², polymère acrylique Acronal LA471S de BASF | 18 g/m², polymère acrylique Primai E1845 de Rhom&Haas | 15 g/m², copolymère Styrène-butadiène ' Latexia PE 1161 de RAISIO | 12g/m², polymère acrylique Primal E , 1845 de Rhom& Haas | 30 g/m², copolymère Styrène-butadiène Latexia PE 1161 de RAISIO |
| Quantité en sec de la cire scellable | 0 | 0 | 0 | 0 | 3 g/m² | 0 | 20g/m² |
| Grammage du matériau (g/m²) | 80 | 80 | 80 | 80 | 80 | 80 | 80 |
| Résistance à la déchirure amorcée (mN) | 1855 | 4100 | 4300 | 2650 | 2350 | 1900 | 1920 |
| Porosité Bendtsen (ml/min) | 700 | 800 | 900 | 660 | 550 | 240 | 400 |
| Diamètre de pores maxi (µm) | 26 | 24 | 26 | 25 | 22 | 18 | 17 |
| BFE (%) | 96% | 96% | 95% | 97% | 98% | 99,5% | 99% |
| Compatibilité procédé de stérilisation | Gaz, irradiation, vapeur | Gaz, irradiation, vapeur | Gaz, irradiation, vapeur | Gaz, irradiation, vapeur | Gaz, irradiation, vapeur | Gaz, irradiation, vapeur | Gaz, irradiation, plasma |
| Observations | Déchirure insuffisante | | | | | | |

## Revendications

1. Matériau d'emballage de stérilisation pour les dispositifs médicaux devant être stérilisés, le matériau étant sous forme d'une feuille fibreuse formée d'une seule couche de fibres comprenant des fibres de polyamide, ladite feuille ayant un grammage inférieur à 100 g/m², mesuré selon la norme internationale ISO 536, une résistance moyenne à la déchirure amorcée, mesurée selon la nonne européenne EN 21974, supérieure ou égale à 1900 mN et une efficacité en filtration bactérienne BFE supérieure ou égale à 95 %, déterminée selon la méthode publiée par l'association européenne EDANA sous la référence 180.0-89 de février 1996, ladite feuille fibreuse comportant en masse des fibres de cellulose raffinées et un liant papetier ou comportant des fibres cellulosiques sous forme d'une pâte fluff.

2. Matériau selon la revendication 1, **caractérisé en ce que** ladite feuille a un grammage inférieur ou égal à 90 g/m², mesuré selon la norme internationale ISO 536.

3. Matériau selon l'une quelconque des revendications 1 à 2, **caractérisé en ce que** les fibres de polyamide sont choisies parmi les fibres de polyamide 6 et de polyamide 6-6.

4. Matériau selon l'une quelconque des revendications 1 à 3, **caractérisé en ce que** lesdites fibres de polyamide ont une longueur supérieure ou égale à 5mm.

5. Matériau selon l'une quelconque des revendications 1 à 4, **caractérisé en ce que** lesdites fibres de polyamide ont un titre moyen supérieur à 1,5 dtex.

6. Matériau selon l'une quelconque des revendications 1 à 5, **caractérisé en ce que** lesdites fibres de polyamide comportent des anti-oxydants leur conférant une résistance à l'irradiation.

7. Matériau selon l'une quelconque des revendications 1 à 6, **caractérisé en ce que** lesdites fibres de polyamide sont des fibres coupées.

8. Matériau selon l'une des revendications précédentes, **caractérisé en ce que** ladite feuille fibreuse comporte en masse des fibres de cellulose raffinées entre 25 et 70 degrés Schoepper-Riegler.

9. Matériau selon l'une quelconque des revendications 1 à 8, **caractérisé en ce que** ladite feuille fibreuse comprend une quantité de fibres de polyamide supérieure à 10 % en poids par rapport au poids total en fibres, de préférence au moins 15% en poids.

10. Matériau selon l'une quelconque des revendications 1 à 7, **caractérisé en ce que** ladite feuille fibreuse comporte comme fibres essentiellement des fibres synthétiques.

11. Matériau selon la revendication 10, **caractérisé en ce que** les fibres synthétiques comportent au moins 50%, de préférence au moins 60% de fibres de polyamide.

12. Matériau selon l'une quelconque des revendications 1 à 11, **caractérisé en ce que** ladite feuille fibreuse comporte en surface un liant, en particulier un liant polymère de base acrylique ou un copolymère styrène-butadiène.

13. Matériau selon la revendication précédente, **caractérisé en ce que** la quantité dudit liant est comprise entre 10 et 35 g/m² en poids sec.

14. Matériau selon l'une des revendications précédentes, **caractérisé en ce qu'**il a été assoupli, en particulier (micro)crêpé ou flexé.

15. Matériau selon l'une des revendications précédentes, **caractérisé en ce qu'**il comporte un produit de scellage sur au moins l'une de ses faces.

16. Emballage de stérilisation comprenant un matériau selon l'une des revendications précédentes.

## Patentansprüche

1. Sterilisationsverpackungsmaterial für medizinische Vorrichtungen, bevor sie sterilisiert werden, wobei das Material in der Form einer faserigen Bahn vorliegt, die aus einer einzelnen Faserschicht gebildet ist, die Polyamidfasern umfasst, wobei die Bahn ein Flächengewicht unterhalb von 100 g/m², gemessen gemäß der internationalen Norm ISO 536, einen mittleren Widerstand gegen Einreißen, gemessen nach der europäischen Norm EN 21974, oberhalb von oder gleich 1900 mN und einen bakteriellen Filtrationswirkungsgrad BFE oberhalb von oder gleich 95%, bestimmt gemäß dem vom Europäischen Verband EDANA unter der Referenz 180.0-89 im Februar 1996 veröffentlichten Verfahren, aufweist, wobei die faserige Bahn eine Masse von verfeinerten Zellulosefasern und ein Papierherstellungsbindemittel umfasst oder Zellulosefasern in der Form eines Flockenzellstoffs umfasst.

2. Material nach Anspruch 1, **gekennzeichnet dadurch, dass** die Bahn ein Flächengewicht unterhalb von oder gleich 90 g/m², gemessen nach der internationalen Norm ISO 536, aufweist.

3. Material nach einem der Ansprüche 1 bis 2, **gekennzeichnet dadurch, dass** die Polyamidfasern ausgewählt sind aus Fasern von Polyamid 6 und Polyamid 6-6.

4. Material nach einem der Ansprüche 1 bis 3, **gekennzeichnet dadurch, dass** die Polyamidfasern eine Länge oberhalb von oder gleich 5 mm aufweisen.

5. Material nach einem der Ansprüche 1 bis 4, **gekennzeichnet dadurch, dass** die Polyamidfasern einen mittleren Titer oberhalb von 1,5 dtex aufweisen.

6. Material nach einem der Ansprüche 1 bis 5, **gekennzeichnet dadurch, dass** die Polyamidfasern Antioxidanzien umfassen, die ihnen einen Bestrahlungswiderstand verleihen.

7. Material nach einem der Ansprüche 1 bis 6, **gekennzeichnet dadurch, dass** die Polyamidfasern geschnittene Fasern sind.

8. Material nach einem der vorhergehenden Ansprüche, **gekennzeichnet dadurch, dass** die faserige Bahn als Masse Zellulosefasern umfasst, die zwischen 25 und 70 Grad Schopper-Riegler verfeinert sind.

9. Material nach einem der Ansprüche 1 bis 8, **gekennzeichnet dadurch, dass** die faserige Bahn einen Anteil an Polyamidfasern oberhalb von 10 Gew.-% bezüglich des Gesamtgewichts an Fasern, vorzugsweise wenigstens 15 Gew.-%, aufweist.

10. Material nach einem der Ansprüche 1 bis 7, **gekennzeichnet dadurch, dass** die faserige Bahn als Fasern im Wesentlichen synthetische Fasern umfasst.

11. Material nach Anspruch 10, **gekennzeichnet dadurch, dass** die synthetischen Fasern wenigstens 50%, vorzugsweise wenigstens 60%, Polyamidfasern umfassen.

12. Material nach einem der Ansprüche 1 bis 11, **gekennzeichnet dadurch, dass** die faserige Bahn oberflächlich ein Bindemittel, insbesondere ein Polymerbindemittel auf Acrylbasis oder ein Copolymer Styren-Butadien, umfasst.

13. Material nach dem vorhergehenden Anspruch, **gekennzeichnet dadurch, dass** der Anteil des Bindemittels zwischen 10 und 35 g/m² im Trockengewicht liegt.

14. Material nach einem der vorhergehenden Ansprüche, **gekennzeichnet dadurch, dass** es weichgemacht ist, insbesondere (mikro-)gekreppt oder geflext ist.

15. Material nach einem der vorhergehenden Ansprüche, **gekennzeichnet dadurch, dass** es ein Versiegelungserzeugnis auf wenigstens einer seiner Flächen aufweist.

16. Sterilisationsverpackung, die ein Material nach einem der vorhergehenden Ansprüche umfasst.

## Claims

1. A sterilization packaging material for medical devices awaiting sterilization, the material being in the form of a monolayer fibrous sheet comprising polyamide fibers, said sheet having a grammage of less than 100 g/m², measured according to international standard ISO 536, a mean initial tear strength, measured according to European Standard EN 21974, higher than or equal to 1900 mN, and a bacterial filtration efficiency (BFE) higher than or equal to 95%, determined according to the method published by the European Association EDANA under the reference 180.0-89 of February 1996, said fibrous sheet including, in bulk, refined cellulose fibers and a paper binder or including, as cellulose fibers, a fluff pulp.

2. The material as claimed in claim 1, **characterized in that** said sheet has a grammage lower than or equal to 90 g/m² measured according to the international standard ISO 536.

3. The material as claimed in any one of claims 1 to 2, **characterized in that** the polyamide fibers are chosen from polyamide-6 and polyamide-6,6 fibers.

4. The material as claimed in any one of claims 1 to 3, **characterized in that** said polyamide fibers have a length higher than or equal to 5 mm.

5. The material as claimed in any one of claims 1 to 4, **characterized in that** said polyamide fibers have a mean linear density greater than 1.5 dtex.

6. The material as claimed in any one of claims 1 to 5, **characterized in that** said polyamide fibers include antioxidants giving them irradiation resistance.

7. The material as claimed in any one of claims 1 to 6, **characterized in that** said polyamide fibers are chopped fibers.

8. The material as claimed in any one of the preceding claims, **characterized in that** said fibrous sheet includes, in bulk, refined cellulose fibers to between 25 and 70 degrees Schoepper-Riegler.

9. The material as claimed in any one of claims 1 to 8, **characterized in that** said fibrous sheet comprises an amount of polyamide fibers of greater than 10% by weight, relative to the total weight of fibers, preferably at least 15% by weight.

10. The material as claimed in any one of claims 1 to 7, **characterized in that** said fibrous sheet comprises, as fibers, essentially only synthetic fibers.

11. The material as claimed in claim 10, **characterized in that** the synthetic fibers comprise at least 50%, preferably at least 60 % of polyamide fibers.

12. The material as claimed in any one of claims 1 to 11, **characterized in that** said fibrous sheet includes, on the surface, a binder, in particular an acrylic-based polymer binder or a styrene-butadiene copolymer.

13. The material as claimed in the preceding claim, **characterized in that** the amount of said binder is between 10 and 35 g/m² by dry weight.

14. The material as claimed in any one of the preceding claims, **characterized in that** it has been softened, in particular (micro) creped or flexed.

15. The material as claimed in any one of the preceding claims, **characterized in that** it includes a sealant on at least one of its faces.

16. A sterilization package comprising material as claimed in any one of the preceding claims.
